# EUROPÄISCHE PATENTANMELDUNG

(11) **EP 0 945 712 A1**
(43) Veröffentlichungstag der Anmeldung: **29.09.1999**
(21) Anmeldenummer: 99104484.3
(22) Anmeldetag: 05.03.1999
(51) Int. Cl.: G01F 1/66, A61B 5/087

(54) **Vorrichtung zum Messen eines Gasflusses**

(30) Priorität: 25.03.1998 SE 9801007
(71) Anmelder: Siemens-Elema AB, 171 95 Solna (SE)
(72) Erfinder: Wallen, Lars, 16360 Spanga (SE); Arvidsson, Carl-Erik, 17139 Solna (SE)

(57) **Zusammenfassung**

Die Erfindung betrifft eine Vorrichtung zum Messen eines Gasflusses umfassend eine Meßkammer und eine an der Meßkammer über Öffnungen der Meßkammer anschließbare Ultraschallsende- und Empfangseinheit, die mit Sende- und Empfangsköpfen versehen ist. Die Köpfe sind gegen die Öffnungen der Meßkammer gerichtet, wobei zwischen der Meßkammer und den Sende- und Empfangsköpfen Membranen angeordnet sind, die duchlässig für Ultraschallwellen, aber weitgehend undurchlässig für Feuchtigkeit und Bakterien sind. Um eine Meßvorrichtung dieser Art zu erhalten, bei der der Ultraschallsende- und Empfangseinheit maximale akustische Energie zugeführt wird, gleichzeitig damit, daß Feuchtigkeit und Bakterien von den Einheiten ferngehalten werden, wird erfindungsgemäß vorgeschlagen, daß die Membranen (11, 12, 25, 28) dicht an den Sende- und Empfangsköpfen (7, 8, 22, 23) austauschbar angeordnet sind.

## Beschreibung

Die Erfindung betrifft eine Vorrichtung zum Messen eines Gasflusses, umfassend eine Meßkammer und eine an der Meßkammer über den Öffnungen der Meßkammer anschließbare Ultraschallsende- und Empfangseinheit, die mit Sende- und Empfangsköpfen versehen ist, die gegen die Öffnungen der Meßkammer gerichtet sind, wobei zwischen der Meßkammer und den Sende- und Empfangsköpfen Membranen angeordnet sind, die für Ultraschallwellen durchlässig, aber weitgehend undurchlässig für Feuchtigkeit und Bakterien sind.

In der DE-C1-4 222 286 ist ein Ultraschallflußmesser der obengenannten Art beschrieben, in dem der Sender und der Empfänger mit Abstand voneinander entlang einer Meßstrecke angeordnet sind. Die Meßstrecke verläuft schräg im Vergleich zur Achse einer rohrförmigen Meßkammer, durch die das Medium, dessen Flußgeschwindigkeit bestimmt werden soll, strömt. Der Flußmesser ist ein sogenanntes Spirometer zur Bestimmung der Lungenkapazität der Patienten. Um dabei die Hygiene sicherzustellen, wird in Verbindung mit jedem neuen Patienten in die Meßkammer ein steriles Einsatzrohr eingeführt. Das sterile Rohr ist mit Meßfenstern versehen, die so angepaßt sind, daß sie über den Öffnungen in der Meßkammer liegen. In den Meßfenstern sind für Ultraschallsignale durchlässige, aber für Feuchtigkeit und Bakterien undurchlässige Membranen angeordnet, so daß die Ultraschallsignale entlang der Meßstrecke durch das sterile Einsatzrohr passieren können. Hierdurch wird vermieden, daß das Krankenhauspersonal nach jeder Verwendung den Flußmesser autoklavieren muß, da, insbesondere die Ultraschallsende- und Empfangseinheit empfindliche Teile in dem Flußmesser sind. Die Membranen, die mit Abstand zu den Ultraschallsende- und Empfangsköpfen angeordnet sind, können in Verbindung mit dem bekannten Ultraschallflußmesser in einem Beispiel aus Schaumgummi und in einem weiteren Beispiel aus einer Mylarfolie bestehen. Damit die Ultraschallsignale die Sende- bzw. Empfangseinheit erreichen können, müssen die Signale in Verbindung mit dem ersten Beispiel zunächst die verhältnismäßig dicke Schaumgummimembran und danach einen verhältnismäßig großen Luftspalt passieren. Dieser Übergang von einer verhältnismäßig dicken Membran zu einem verhältnismäßig großen Luftspalt kann zu einer hohen akustischen Impedanz, d.h. einer hohen Schallwellenreflexion führen. Dies kann zu verhältnismäßig großen akustischen Verlusten führen, so daß ein nicht annehmbar niedriges Schallsignal die Sende- bzw. Empfangsköpfe erreicht. In Verbindung damit, daß eine Mylarfolie benutzt wird, ist aufgrund dessen, daß sie extrem dünn ist, eine verhältnismäßig niedrige akustische Impedanz gegeben, wobei ein annehmbares Schallsignal die erwähnten Köpfe erreichen kann. Der Nachteil mit Mylarfolien, die auf beschriebene Weise angebracht sind, ist, daß sie derart dünn und damit derart empfindlich sind, daß sie nicht immer die mechanische Belastung, der sie ausgesetzt werden, wenn ein Überdruck in der Meßkammer entsteht, vertragen, wodurch die Folien leicht reißen können.

Der Erfindung liegt die Aufgabe zugrunde, eine Vorrichtung der eingangs genannten Art zu schaffen, bei der der Ultraschallsende- und Empfangseinheit maximale akustische Energie zugeführt wird gleichzeitig damit, daß Feuchtigkeit und Keime von den Einheiten fern gehalten werden.

Diese Aufgabe ist erfindungsgemäß dadurch gelöst, daß die Membranen dicht an den Sende- und Empfangsköpfen austauschbar angeordnet sind. Dadurch, daß die Membranen austauschbar sind, können sie nach einer Untersuchung entfernt und in Verbindung mit einem neuen Patienten durch neue Membranen ersetzt werden. Als Alternative können die Membranen eventuell gereinigt und wiederverwendet werden. Dadurch, daß die Membranen dicht an den Sende- und Empfangsköpfen angeordnet sind, wird die akustische Impedanz effektiv verringert, wobei den erwähnten Köpfen mehr akustische Energie zugeführt wird.

Durch die Plazierung der Membranen können sehr dünne Membranen aus z.B. Metall oder Polymer verwendet werden, da sie in dieser Lage nicht mehr einer mechanischen Belastung ausgesetzt werden. Auch eine dickere Membran aus z.B. Schaumgummi kann nun mit nahezu beibehaltener guten akustischen Energiezufuhr an den Sende- und Empfangsköpfen verwendet werden. Der Vorteil einer dicken Membran ist deren gute Haltbarkeit.

In der Fachzeitschrift "IEEE Transactions on Sonics and Ultrasonics" Vol. SU-31, No. 2, vom März 1984 unter dem Titel "Impedance-Matched Metallurgically Sealed Transducers" (Seite 101 bis 104) ist ein robuster Ultraschallsensor beschrieben, dessen Kopf mit einer verhältnismäßig dicken Membran versehen ist, die mit dem Sensor fest verbunden ist und für Ultraschallwellen durchlässig, aber für Feuchtigkeit und Bakterien undurchlässig ist. Vor der Membran ist eine dünne Plastikfolie angebracht, die ausschließlich dafür vorgesehen ist, die akustische Impedanz, die sonst verhältnismäßig hoch ist, wenn eine Metallmembran der genannten Art verwendet wird, zu verringern. Es ist in dem Artikel nicht erwähnt, daß die dünne Plastikfolie verhindert, daß Feuchtigkeit und Bakterien die Metallmembran erreichen können. Daher ist es trotz der Metallmembran und der Plastikfolie notwendig, bei Benutzung des hier beschriebenen Sensors in Verbindung mit einem Ultraschallflußmesser diesen Sensor vor jedem neuen Patienten zu autoklavieren, da Feuchtigkeit und Bakterien auf der Metallmembran vorhanden sein können. Ein regelmäßiges Autoklavieren des Sensors ergibt eine verkürzte Lebensdauer.

In einer vorteilhaften Ausbildung der Vorrichtung nach der Erfindung wird vorgeschlagen, daß jeder Sende- und Empfangskopf gegen die jeweilige Membran drückt. Bei einer derartigen Ausführung sind die Membranen in der Meßkammer z.B. abnehmbar befestigt. Wenn nun die Köpfe gegen die jeweilige Membran gedrückt werden, wird beinahe die gesamte Luft zwischen der Membran und dem Kopf zur Seite gedrückt, so daß die Köpfe dicht an die jeweilige Membran zu liegen kommen, wodurch eine weitere Verringerung der akustischen Impedanz erreicht werden kann.

In einer weiteren vorteilhaften Ausbildung der Vorrichtung nach der Erfindung wird vorgeschlagen, daß die Membran am Sende- und Empfangskopf angebracht ist. Auf diese Weise können die Köpfe des Senders und des Empfängers mit Membranen versehen werden, bevor sie an die Meßkammer angeschlossen werden. Nach einer Untersuchung wird der Sender und der Empfänger von der Meßkammer gelöst, wobei die Membranen ausgewechselt werden und Sender und Empfänger in Verbindung mit einem neuen Patienten verwendet werden.

In einer weiteren vorteilhaften Ausführungsform der Vorrichtung nach der Erfindung wird vorgeschlagen, daß jeder Sende- und Empfangskopf wenigstens über ein Teil seiner Oberfläche mit der Membran mittels eines Adhesivs verbunden ist. Hier ist die Membran auf geeignete Weise mit einer Adhesivschicht versehen. Beim Austauschen der Membran wird die Adhesivschicht vom Kopf entfernt. Dadurch, daß die Membran bei einer solchen Ausführungsform eine mechanische Verbindung mit dem Kopf hat, wird die akustische Impedanz nochmals verringert. Es ist hier auch eine mechanische Stabilität der Membran gegeben.

Die Erfindung ist nachfolgend anhand mehrerer in den Zeichnungen dargestellter Ausführungsbeispiele näher erläutert. Es zeigen:
- FIG. 1: eine Prinzipskizze einer Meßvorrichtung nach der Erfindung in einem Längsschnitt,
- FIG. 2: eine Prinzipskizze einer weiteren Meßvorrichtung nach der Erfindung in einem Längsschnitt,
- FIG. 3: eine Prinzipskizze einer weiteren Meßvorrichtung nach der Erfindung, auch diese in einem Längsschnitt,
- FIG. 4: den Aufbau eines Teils einer Meßvorrichtung nach der FIG. 3 und
- FIG. 5: den Aufbau desselben Teils der Meßvorrichtung wie in der FIG. 4, aber in einer weiteren Ausführungsform.

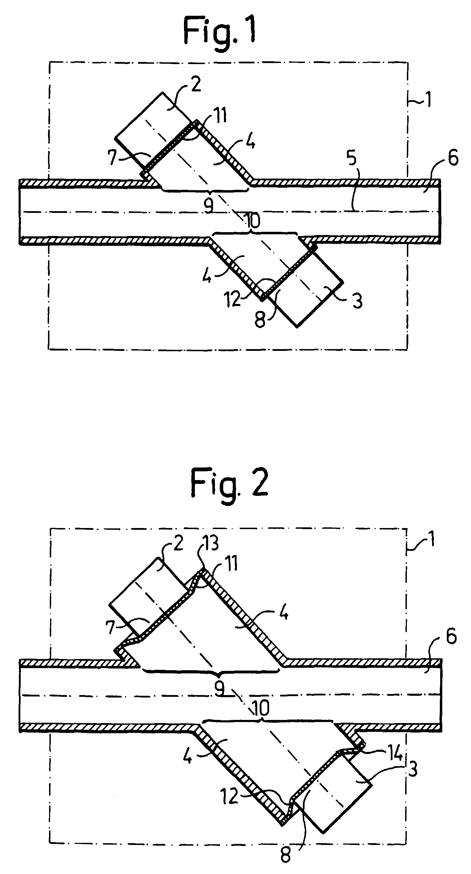

In der FIG. 1 ist ein Ultraschallflußmesser 1, in dem ein Ultraschallsender 2 und ein Ultraschallempfänger 3 mit Abstand voneinander entlang einem Kanal 4, der als Meßstrecke dient, angeordnet ist, schematisch gezeigt. Der Kanal 4 verläuft schräg im Vergleich zur Achse 5 einer rohrförmigen Meßkammer 6, durch die das Medium, dessen Flußgeschwindigkeit bestimmt werden soll, strömt. Der Flußmesser ist ein sogenanntes Spirometer zur Bestimmung der Lungenkapazität des Patienten. Der Ultraschallsender 2 und der Empfänger 3 sind mit Sende- und Empfangsköpfen 7, 8 versehen, die gegen Öffnungen 9, 10 in der Meßkammer 6, durch die die Meßstrecke 4 verläuft, gerichtet sind. Ein Ultraschallflußmesser 1 dieser Art ist in der WO 94/28790 gezeigt und beschrieben. In dem Ultraschallflußmesser 1 nach der Erfindung sind Membranen 11, 12 vorgesehen, die für Ultraschallwellen durchlässig, aber für Feuchtigkeit und Bakterien weitgehend undurchlässig sind und die, wie die FIG. darstellen soll, dicht an den Sende- und Empfangsköpfen 7, 8 angeordnet sind. In diesem Ausführungsbeispiel ist der Innendurchmesser des Kanals 4 etwa genau so groß wie der Außendurchmesser der Sende- und Empfangsköpfe 7, 8. Bei einer derartigen Ausführungsform ist es von Vorteil, die Membranen 11, 12 direkt an den Sende- bzw. Empfangsköpfen 7, 8, z.B. mit Hilfe eines Adhesivs, anzubringen. Durch Applizieren der Membranen 11, 12 aus Metall oder aus einem Polymer dicht an den jeweiligen Sende- und Empfangskopf 7, 8, ist die akustische Impedanz vergleichsweise gering, wobei den Köpfen 7, 8 verhältnismäßig viel akustische Energie zugeführt werden kann. Bei einer derartigen Applizierung können verhältnismäßig dicke Membranen angebracht werden, ohne daß die akustische Energie, die den jeweiligen Kopf 7, 8 erreicht, nennenswert verringert wird. Eine sehr kleine akustische Impedanz wird erhalten, wenn eine dünne Membran verwendet wird. Dadurch, daß die Membranen 11, 12 in dem beschriebenen Ausführungsbeispiel mit dem jeweiligen Kopf 7, 8 über ein Adhesiv verbunden sind, werden die Membranen 11, 12 mit den Köpfen 7, 8 in mechanischen Kontakt gebracht, wobei eine weitere Verringerung der akustischen Impedanz erreicht wird. Da die Membranen 11, 12 austauschbar sind, werden sie nach einer Untersuchung entfernt und vor jedem neuen Patienten werden neue Membranen 11, 12 appliziert. Als eine Alternative können die Membranen 11, 12 autoklaviert und wiederverwendet werden.

In der FIG. 2 ist ein Ultraschallflußmesser 1 gezeigt, der sich von dem in Verbindung mit FIG. 1 beschriebenen Ultraschallflußmesser 1 unterscheidet, indem der Kanal 4 einen größeren Innendurchmesser als die Außendurchmesser des jeweiligen Sende- und Empfangskopfes 7, 8, aufweist. Ein weiterer Unterschied besteht darin, daß die Membranen 11, 12 an den freien Enden 13, 14 des Kanals 4 abnehmbar angebracht sind. Durch Drücken des jeweiligen Kopfes 7, 8 des Senders 2 und des Empfängers 3 gegen die jeweilige Membran 11, 12, wird nahezu die ganze Luft zwischen den Membranen 11, 12 und den Köpfen 7, 8 weggedrückt, wobei die Köpfe 7, 8 dicht gegen die Membranen 11, 12 zu liegen kommen. Bei einem solchen Ausführungsbeispiel brauchen die Membranen 11, 12 nicht notwendigerweise mit einem Adhesiv versehen werden. In Verbindung mit dem in dieser FIG. beschriebenen Ausführungsbeispiel können der Ultraschallsender 2, der Ultraschallempfänger 3 sowie die Membranen 11, 12 nach jeder Untersuchung entfernt und vor einem neuen Patienten neue Membranen 11, 12 am jeweiligen Kopf 7, 8 appliziert werden. Danach werden, wie bereits beschrieben, der Sender und der Empfänger wieder gegen die jeweiligen Membranen 11, 12 gedrückt und in dieser Lage arretiert.

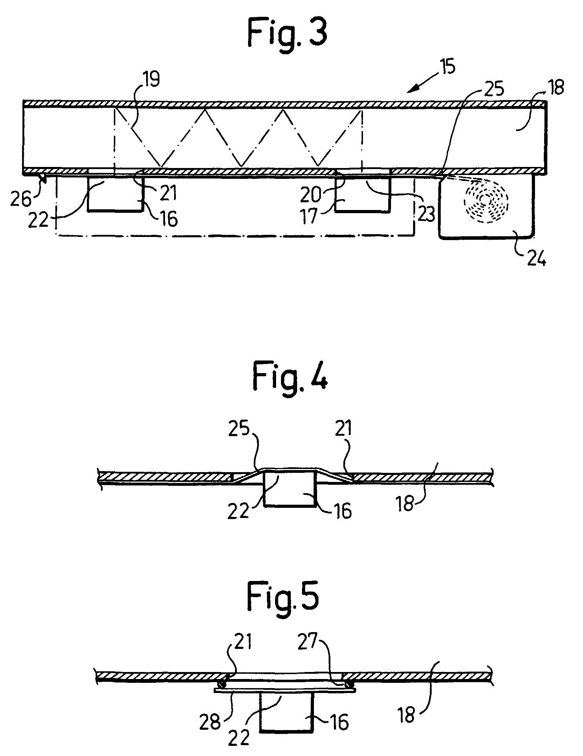

In der FIG. 3 ist ein weiterer, schematisch gezeichneter Ultraschallflußmesser 15 gezeigt. In Zusammenhang mit diesem Flußmesser ist der Sender 16 und der Empfänger 17 an derselben Seite der langgestreckten Meßkammer 18 angeordnet. Hier kann der Sender 16 ein akustisches Signal, hier mit 19 bezeichnet, abgeben, das über eine Anzahl Reflexionen an den Wänden der Meßkammer 18 durch eine durch die Meßkammer 18 strömende Gasmischung hindurch übertragen wird, um danach auf den Empfänger 17 aufzutreffen, der nun das übertragene akustische Signal entgegennimmt. Ein solcher Ultraschallflußmesser 15 ist in der schwedischen noch nicht veröffentlichten Patentanmeldung mit der Nr. 9701477-3 näher beschrieben. Der Durchmesser derjenigen Öffnungen 20, 21, die an der Meßkammer 18 angeordnet und die für den Sender 16 und den Empfänger 17 vorgesehen sind, sind nach der Erfindung etwa genau so groß wie der Außendurchmesser des jeweiligen Kopfes 22, 23 des Senders 16 und des Empfängers 17. In diesem Ausführungsbeispiel ist an der Meßkammer 18 eine Halterung 24 angeordnet, beinhaltend einen aufgerollten Vorrat mit bandförmiger Membran 25. Vor einer Untersuchung wird die bandförmige Membran 25 so weit aus der Halterung herausgerollt, daß sie die beiden Öffnungen 20, 21 der Meßkammer 18 abdeckt. Die Membran 25 kann wenigstens auf der Seite, die gegen die Außenwand der Meßkammer 18 zu liegen kommt, mit einem Adhesiv versehen sein, um damit die Membran schnell und einfach applizieren zu können. Danach wird der Sender 16 und der Empfänger 17 gegen die Membran 25 an den jeweiligen Öffnungen 21, 22 angebracht. Die Membran 25 kann auch vorzugsweise auf der der Meßkammer 18 entgegengerichteten Seite mit einem Adhesiv versehen sein. Hierdurch ist ein sehr guter Kontakt zwischen den Köpfen 22, 23 und der Membran 25 gegeben. Die Außenwand der Meßkammer 18 ist mit einem Abreißteil 26 für die Membran 22 versehen. Nach einer Untersuchung wird der Sender 16 und der Empfänger entfernt. Die Membran 25 wird nun von der Außenwand der Meßkammer 18 gelöst. Danach wird vor der nächsten Untersuchung eine weitere Strecke mit Membran 25 aus der Halterung 25 herausgezogen, bis die Membran 25 die Öffnungen 21, 22 abdeckt, wobei die Membran 25, die bei der vorhergehenden Messung verwendet wurden mit Hilfe des Abreißteils 26 abgerissen wird. Danach wird der Sender 16 und der Empfänger 17 wieder gegen die Membran 25 appliziert.

In der FIG. 4 soll gezeigt werden, daß die Öffnungen 20, 21 größer als der Außendurchmesser des jeweiligen Kopfes 22,23 des Senders 16 und des Empfängers 17 sein können. Bei einem solchen Ausführungsbeispiel kann, wie es in Verbindung mit der FIG. 2 beschrieben worden ist, der Sender 16 bzw. der Empfänger 17 gegen die Membran gedrückt werden, um somit einen Luftspalt zwischen den genannten Teilen auf ein Minimum zu reduzieren. In Verbindung mit diesem Beispiel braucht die Seite der Membran 25, die gegen den Sender 16 bzw. den Empfänger 17 gewandt ist, nicht notwendigerweise mit einem Adhesiv versehen sein. In der FIG. ist lediglich die Öffnung 21 mit dem Sender 16 gezeigt.

In der FIG. 5 soll dargestellt werden, daß zwischen der Außenwand der Meßkammer 18 und der Membran 28 ein Dichtungsring anbringbar ist. In einem solchen Ausführungsbeispiel ist es von Vorteil, wenn jede Öffnung 20, 21 der Meßkammer 18 mit jeweils einer Membran 28 versehen ist. Durch den Dichtungsring 27 ist eine sehr gute Dichtung zwischen dem Inneren der Meßkammer 18 und der Atmosphäre gegeben. In dieser FIG. ist nur die Öffnung 21 mit dem Sender 16 gezeigt. Die Öffnung 20 und der Empfänger 17 weisen vorzugsweise die gleiche Form auf.

## Patentansprüche

1. Vorrichtung zum Messen eines Gasflusses umfassend eine Meßkammer und eine an der Meßkammer über Öffnungen der Meßkammer anschließbare Ultraschallsende- und Empfangseinheit, die mit Sende- und Empfangsköpfen versehen ist, die gegen die Öffnungen der Meßkammer gerichtet sind, wobei zwischen der Meßkammer und den Sende- und Empfangsköpfen Membranen angeordnet sind, die durchlässig für Ultraschallwellen, aber weitgehend undurchlässig für Feuchtigkeit und Bakterien sind, **dadurch gekennzeichnet,** daß die Membranen (11, 12, 25, 28) dicht an den Sende- und Empfangsköpfen (7, 8, 22, 23) austauschbar angeordnet sind.

2. Vorrichtung nach Anspruch 1, **dadurch gekennzeichnet,** daß jeder Sende- und Empfangskopf (7, 8, 22, 23) gegen die jeweilige Membran (11, 12, 25, 28) drückt.

3. Vorrichtung nach Anspruch 1 oder 2, **dadurch gekennzeichnet,** daß die Membran (11, 12, 25, 28) am Sende- und Empfangskopf (7, 8, 22, 23) angebracht ist.

4. Vorrichtung nach einem der Ansprüche 1 bis 3, **dadurch gekennzeichnet,** daß jeder Sende- und Empfangskopf (7, 8, 22, 23) wenigstens über ein Teil seiner Oberfläche mit der Membran (11, 12, 25, 28) mittels eines Adhesivs verbunden ist.
